(19) Europäisches Patentamt
European Patent Office
Office européen des brevets

(11) Veröffentlichungsnummer: **0 001 079**
**B1**

(12) # EUROPÄISCHE PATENTSCHRIFT

(45) Veröffentlichungstag der Patentschrift: 08.07.81

(21) Anmeldenummer: 78100761.2

(22) Anmeldetag: 28.08.78

(51) Int. Cl.³: A 61 K 7/06, A 61 K 7/08, A 61 K 7/48

(54) **Kosmetisches Präparat mit antiseborrhoischer Wirkung, Verfahren zu dessen Herstellung und dessen Verwendung.**

(30) Priorität: 14.09.77 CH 11213/77

(43) Veröffentlichungstag der Anmeldung:
21.03.79 Patentblatt 79/6

(45) Bekanntmachung des Hinweises auf die Patenterteilung:
08.07.81 Patentblatt 81/27

(84) Benannte Vertragsstaaten:
BE CH DE FR GB NL

(56) Entgegenhaltungen:
FR - A - 1 264 810
GB - A - 1 337 467
US - A - 4 007 261

DICTIONNAIRE VIDAL, 1968, L. VIDAL, Office de vulgarisation pharmaceutique, Paris,
*Seite 1177,

HANDBUCH DER KOSMETIKA UND REICHSTOFFE, Band I "Die kosmetischen Grundstoffe", Zweite Auflage, 1969, Hüthig Verlag, H. JANISTYN, Heidelberg
*Seite 1077, Seite 1078

(73) Patentinhaber: F. HOFFMANN-LA ROCHE & CO. Aktiengesellschaft
CH-4002 Basel (CH)

(72) Erfinder: Bauer, Robert
Talmattstrasse 88
CH-4126 Bettingen (CH)
Erfinder: Hostettler, Hans Ulrich, Dr.
Weidenhofstrasse 28
CH-4144 Arlesheim (CH)

(74) Vertreter: Urech, Peter, Dr., et al,
Grenzacherstrasse 124, Postfach 601
CH-4002 Basel (CH)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 079 B1

Kosmetisches Präparat mit antiseborrhoischer Wirkung, Verfahren zu dessen Herstellung und dessen Verwendung.

Die Erfindung betrifft ein kosmetisches Präparat, und zwar ein Pyridoxintripalmitat enthaltendes Präparat mit antiseborrhoischer Wirkung, das dadurch gekennzeichnet ist, dass es als Solubilisator ein Aminoxyd der allgemeinen Formel

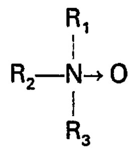
I

worin $R_1$ Alkyl mit 16—20 Kohlenstoffatomen und
$R_2$ und $R_3$ Hydroxyäthyl oder Methyl bedeuten, enthält.

Crèmes, die das Tripalmitat des Pyridoxins enthalten, sind beispielsweise aus Seifen-Oele-Fette-Wachse *26*, 819 (1959) bekannt. Bei Anwendung dieser Crèmes wurde frühestens nach 2 Wochen eine hautpflegende und schwach talgdrüsenhemmende Wirkung an Probanden beobachtet.

Auch im Handbuch der Kosmetika und Riechstoffe, Band 1, S. 1077—1078 wird das Pyridoxintripalmitat, seine physikalischen Eigenschaften und seine antiseborrhoische Wirkung erwähnt.

Aus der britischen Patentschrift Nr. 1.337.467 ist das Dimethyldodecylaminoxyd als organisches Netzmittel für wässrige Mittel, z.B. wässrige Reinigungsmittel, bekannt geworden.

Die französische Patentschrift Nr. 1.264.810 beschreibt Haarpflegemittel, die Aminoxyde als Bakterizide und Fungizide enthalten. Als Aminoxyde werden u.a. Bis(hydroxyäthyl)dodecylaminoxyd und Dimethyldodecylaminoxyd erwähnt.

Es wurde nun gefunden, dass eine rasch eintretende talgdrüsenhemmende Wirkung des Pyridoxintripalmitats erzielt werden kann, wenn das erfindungsgemässe Präparat, zur Anwendung gelangt. Ein Fett-bzw Oelgehalt ist beim erfindungsgemäßen Präparat nicht erforderlich.

Die Herstellung derartiger Präparate, die im wesentlichen das Pyridoxintripalmitat in solubilisierter Form enthalten, gelingt mit Hilfe eines Aminoxydes der Formel I, wobei 0,1%—10%, vorzugsweise 0,1%—2% an Aminoxyd verwendet werden. Auf diese Art und Weise lässt sich beispielsweise ein Antifett-Gesichtswasser mit oder ohne Alkoholgehalt, ein Antifett-Shampoo, eine Haarkur gegen fettes Haar oder sogar eine Antifett-Crème herstellen. Bis anhin wurde die Auffassung vertreten, dass derartige Präparate nicht hergestellt werden können, da das Pyridoxintripalmitat nur mit Hilfe einer Oelphase oder Fettphase zu einem kosmetischen Präparat formulierbar sei.

Das Antifett-Gesichtswasser enthält 0—50%, vorzugsweise 0—20% Alkohol. die kosmetischen Präparate enthalten im allgemeinen 0,2—5%, vorzugsweise, 1—2% Pyridoxintripalmitat neben üblichen Trägermaterialien wie beispielsweise p-Hydroxybenzoesäuremethylester, Stearinsäure, Cetylalkohol, Stearylalkohol, Amphisol® (komplexe Verbindung von Alkylphosphat und Diäthanolamin; Lieferant: Givaudan), p-Hydroxybenzoesäurepropylester, Milchsäure, Parfum, Allantoin-N-acetyl-DL-methionat, Menthol, $Al_2(OH)_5Cl$, Natriumchlorid, Texapon® CS Paste (Gemisch von Fettalkoholsulfaten, Polyäthylenglycol-400-monostearat, Zitronensäure, 2,2',4,4'-Tetrahydroxybenzophenon, Weinsäure, usw.

Bevorzugte Aminoxyde der Formel I sind beispielsweise Stearyldimethylaminoxyd, Dimethylcetylaminoxyd, Bis(hydroxyäthyl)-talgfettaminoxyd, Dimethylhexadecylaminoxyd und hydriertes Dimethyltalgfettaminoxyd.

Die talgdrüsenhemmende Wirkung von in den nachstehenden Beispielen beschriebenen Antifett-Gesichtswassern wurde in Anlehnung an die Glasklötzchen-Methode von Schaefer und Kuhn-Bussius, Arch. Klin, exper. Derm. *238*, 429—435 (1970) bestimmt. Es konnte festgestellt werden, dass beispielsweise 2 Stunden nach Anwendung eines nachstehende beschriebenen Antifett-Gesichtswassers die Menge des messbaren Hauttalges um ca. 50% erniedrigt ist.

Die nachstehenden Beispiele erläutern die Erfindung. Alle Temperaturen sind in °C und alle Prozentzahlen in Gewichtsprozent angegeben.

Beispiel 1

*Antifett-Gesichtswasser*

| | |
|---|---|
| Pyridoxintripalmitat | 2,00 g |
| Ammonyx® SO[1)] (Stearyldimethylaminoxyd 25%) | 8,00 g |
| Milchsäure | 0,14 g |
| Almeth (Allantoin-N-acetyl-DL-methionat)[2)] | 1,00 g |

| | |
|---|---|
| Alkohol 95% | 47,38 g |
| Menthol | 0,10 g |
| Wasser | 41,38 g |

40 Wasseranteile werden mit dem ammonyx® auf 75° erwärmt und homogen verrührt. Pyridoxintripalmitat wird bei 80° geschmolzen und unter schnellem Rühren beigefügt. Milchsäure wird mit 1,38 Wasseranteilen vermischt und bei 50° eingegeben, anschliessend Almeth zugegeben. Menthol wird in Alkohol gelöst und bei 40° zugefügt. Danach wird das Gemisch gerührt bis es erkaltet ist.

Beispiel 2

*Antifett-Gesichtswasser*

| | |
|---|---|
| Pyridoxinpalmitat | 2,00 g |
| Ammonyx® CO[1] (Dimethylcetylaminoxyd 30%) | 6,67 g |
| Milchsäure | 1,00 g |
| Chlorhydrol [6] 50% ($Al_2(OH)_5Cl$ | 2,25 g |
| Parfum | 0,20 g |
| Farbstoff | 0,10 g |
| Alkohol 95% | 20,00 g |
| Wasser | 67,78 g |

60 Wasseranteile werden mit dem Ammonyx® auf 75° erwärmt und homogen verruhrt. Pyridoxintripalmitat wird bei 80° geschmolzen und unter schnellem Rühren beigefügt. Milchsäure wird mit 7,78 Wasseranteilen vermischt und bei 50° eingegeben, anschliessend wird Chlorhydrol zugegeben. Parfum und Farbstoff werden im Alkohol gelöst und bei 40° zugefügt. Danach wird das Gemisch gerührt bis es erkaltet ist.

Beispiel 3

Anstelle von Ammonyx® CO in Beispiel 2 wird eine entsprechende Menge Ammonyx® MCO[1] verwendet.

Beispiel 4

*Antifett-Gesichtswasser*

| | |
|---|---|
| Pyridoxintripalmitat | 2,00 g |
| Aromox® T/12[3] (Bis(hydroxyäthyl)-talgfett-aminoxyd 49%) | 4,08 g |
| Zitronensäure | 0,32 g |
| Alkohol 95% | 20,00 g |
| Wasser | 73,60 g |

Wasser und Aromox® werden auf 75° erwärmt und homogen verrührt. Pyridoxintripalmitat wird bei 80° geschmolzen und unter schnellem Rünren beigefügt. Zitronensäure wird in Alkohol gelöst und bei 40° zugegeben. Danach wird das Gemisch gerührt bis es erkaltet ist.

Beispiel 5

Anstelle von Aromox® T/12 in Beispiel 4 wird eine entsprechende Menge Aromox® DM 16 D[3]) (Dimethylhexadecylaminoxyd 39%) verwendet.

Beispiel 6

Anstelle von Aromox® T/12 in Beispiel 4 wird eine entsprechende Menge Aromox® DMHTD[3] (hydriertes Dimethyltalgfettaminoxyd 39%) verwendet.

Beispiel 7

*Antifett-Gesichtswasser*

| | |
|---|---|
| Pyridoxintripalmitat | 2,00 g |
| Aromox® DM 18 D-W[3] (Dimethylstearyl-aminoxyd 25%) | 8,00 g |
| Milchsäure | 0,20 g |
| Natriumchlorid | 0,80 g |
| Alkohol 95% | 20,00 g |
| Farbstoff | 0,10 g |
| Wasser | 68,90 g |

58,90 Wasseranteile werden mit dem Aromox® auf 75° erwärmt und homogen verrührt. Pyridoxintripalmitat wird bei 80° geschmolzen und unter schnellem Rühren beigefügt. Milchsäure wird mit 2 Wasseranteilen vermischt und bei 50° eingegeben. Farbstoff wird in Alkohol gelöst und bei 40° zugefügt. Das Natriumchlorid wird in 8 Wasseranteilen gelöst und zugegeben. Danach wird das Gemisch gerührt bis es erkaltet ist.

Beispiel 8

*Antifett-Gesichtswasser ohne Alkohol*

| | |
|---|---|
| Pyridoxintripalmitat | 2,00 g |
| Ammonyx® SO[1] (Dimethylstearyl-aminoxyd 25%) | 8,00 g |
| Milchsäure | 0,12 g |
| Nipagin® M[4] (p-Hydroxybenzosäure-methylester) | 0,10 g |
| Parfum | 0,10 g |
| Wasser | 89,68 g |

88 Wasseranteile werden mit dem Ammonyx® und Nipagin® auf 75° erwärmt und homogen verrührt. Pyridoxintripalmitat wird bei 80° geschmolzen und unter schnellem Rühren beigefügt. Milchsäure wird mit 1,68 Wasseranteilen vermischt und bei 50° eingegeben. Das Parfum wird bei 45° zugefügt. Danach wird das Gemisch gerührt bis es erkaltet ist.

Beispiel 9

*Antifett-Shampoo*

| | |
|---|---|
| Pyridoxintripalmitat | 2,00 g |
| Texapon® CS Paste[5] (Gemisch von Fettalkoholsulfaten | 33,00 g |
| Ammonyx® SO[1] (vgl. Beispiel 1) | 4,00 g |
| Ammonyx® MCO[1] (vgl. Beispiel 3) | 4,00 g |

| | |
|---|---|
| Cetylalkohol | 0,50 g |
| Stearylalkohol | 0,50 g |
| Polyäthylenglykol-400-monostearat | 1,00 g |
| Zitronensäure 10% ig | 4,40 g |
| Farbstoff 0,2% | 4,00 g |
| Konservierungsmittel | 0,20 g |
| Parfum | 0,40 g |
| Wasser | 46,00 g |

Texapon® CS Paste, Ammonyx® SO u. MCO, Wasser, Cetylalkohol, Stearylalkohol, Polyäthylenglykol-400-monostearat und Konservierungsmittel werden zusammen auf 75° erwärmt und homogen verrührt. Pyridoxintripalmitat wird bei 80° geschmolzen und unter kräftigem Rühren beigefügt. Die Zitronensäurelösung wird bei 50° zugegeben und bei 45° Farbstoff und Parfum zugefügt. Danach wird das Gemisch gerührt bis es erkaltet ist.

Beispiel 10

*Haarkur gegen fettes Haar*

| | |
|---|---|
| Wasser | 69,675 g |
| Ammonyx® SO[1] (Stearyldimethylaminoxyd 25%) | 8,000 g |
| Uvinul® D 50 (2,2',4,4'-Tetrahydroxy-benzophenon) | 0,025 g |
| Pyridoxintripalmitat | 2,000 g |
| Weinsäure pulv. | 0,250 g |
| Polyquart® H 50%[5] (Polyglykol-polyamin-Kondensationsharz) | 1,500 g |
| Wasser | 10,000 g |
| BTC 2125 50%[1] (n-Alkyl-dimethyl-benzyl-ammonium-chlorid, n-Alkyl-dimethyl-äthyl-benzyl-ammoniumchlorid) | 0,200 g |
| Parfum | 0,200 g |
| Farbstoff | 0,150 g |
| NaCl-Lösung 10% g/g | 8,000 g |

69,675 Wasseranteile werden mit dem Ammonyx® auf 75° erwärmt und homogen verrührt. Pyridoxintripalmitat wird bei 80° geschmolzen und unter schenellem Rühren zugegeben. Uvinul® D 50, Weinsäure, Polyquart® H, 10 Wasseranteile und BTC 2125 werden zusammen auf 50° erwärmt und bei 50° dem Ansatz beigefügt. Bei 45° werden nacheinander Parfum, Farbstoff und Natriumchloridlösung zugefügt. Danach wird das Gemisch gerührt bis es erkaltet ist.

Beispiel 11

*Antifett-Crème*

| | |
|---|---|
| Wasser | 84,990 |
| Ammonyx® SO[1] (Stearyldimethylaminoxyd 25%) | 8,000 |

| | |
|---|---|
| Nipagin® M[4]) p-Hydroxybenzoesäure-methylester) | 0,200 |
| Stearinsäure | 2,500 |
| Pyridoxintripalmitat | 2,000 |
| Cetylalkohol | 0,500 |
| Stearylalkohol | 0,500 |
| Amphisol®[7]) (Komplexe Verbindung von Alkylphosphat und Diäthanolamin-Givaudan | 1,000 |
| Nipasol® M [4]) (p-Hydroxybenzoesäure-propylester) | 0,100 |
| Milchsäure | 0,160 |
| Parfum | 0,050 |
| pH-Wert = 6.20 | 100,000 |

83,59 Wasseranteile, Ammonyx® SO u. Nipagin® M werden zusammen auf 75° erwärmt und homogen verrührt. Stearinsäure, Pyridoxintripalmitat, Cetylalkohol, Stearylalkohol, Amphisol® und Nipasol® M werden bei 80° geschmolzen und unter kräftigem Rühren der Wasserphase beigefügt. Danach wird Milchsäure mit 1,40 Wasseranteilen vermischt und bei 50° dem Ansatz zugefügt. Bei 45° wird Parfum zugegeben. Danach wird das Gemisch gerührt bis es erkaltet ist.

*Hersteller der Rohstoffe*

1) Millmaster-Onyx International
2) Nobel Hoechst Chimie
3) AKZO Chemie/Armour Hess Division
4) NIPA Laboratories Ltd.
5) Henkel & Cie GmbH
6) REHEIS CHEMICAL CO.
7) Givaudan S.A., VERNIER

**Patentansprüche**

1. Kosmetisches Präparat mit antiseborrhoischer Wirkung enthaltend Pyridoxintripalmitat, dadurch gekennzeichnet, dass es als Solubilisator ein Aminoxyd der allgemeinen Formel

$$R_2-\underset{R_3}{\overset{R_1}{N}}\rightarrow O \qquad I$$

worin $R_1$ Alkyl mit 16—20 Kohlenstoffatomen und
$R_2$ und $R_3$ Hydroxyäthyl oder Methyl bedeuten, enthält.

2. Präparat gemäss Anspruch 1, dadurch gekennzeichnet, dass es 0,1—10% an einem Aminoxyd der Formel I enthält.

3. Präparat gemäss Anspruch 1, dadurch gekennzeichnet, dass es 0,1%—2% an einem Aminoxyd der Formel I enthält.

4. Präparat gemäss Anspruch 1, dadurch gekennzeichnet, dass es bis zu 50% Alkohol enthält.

5. Präparat gemäss Anspruch 1, dadurch gekennzeichnet, dass es bis zu 20% Alkohol enthält.

6. Präparat gemäss einem der Ansprüche 1—5, dadurch gekennzeichnet, dass es 0,2%—5% Pyridoxintripalmitat enthält.

7. Präparat gemäss einem der Ansprüche 1—5, dadurch gekennzeichnet, dass es 1%—2% Pyridoxintripalmitat enthält.

8. Präparat gemäss einem der Ansprüche 1—7, dadurch gekennzeichnet, dass es Stearyldimethylaminoxyd enthält.

9. Präparat gemäss einem der Ansprüche 1—7, dadurch gekennzeichnet, dass es Dimethylcetylaminoxyd enthält.

10. Präparat gemäss einem der Ansprüche 1—7, dadurch gekennzeichnet, dass es Bis(hydroxyäthyl)-talgfettaminoxyd enthält.

11. Präparat gemäss einem der Ansprüche 1—7, dadurch gekennzeichnet, dass es Dimethylhexadecylaminoxyd enthält.

12. Präparat gemäss einem der Ansprüche 1—7, dadurch gekennzeichnet, dass es hydriertes Dimethyltalgfettaminoxyd enthält.

13. Verfahren zur Herstellung eines kosmetischen Präparates gemäss einem der Ansprüche 1—12, dadurch gekennzeichnet, dass man Pyridoxintripalmitat und eine Verbindung der Formel I in Anspruch 1, gegebenenfalls in Gegenwart von üblichen kosmetischen Hilfsstoffen, unter Erwärmen vermischt.

14. Verwendung eines Präparates gemäss einem der Ansprüche 1—12 als Antifett-Gesichtswasser.

15. Verwendung eines Präparates gemäss einem der Ansprüche 1—12 als Antifett-Shampoo.

16. Verwendung eines Präparates gemäss einem der Ansprüche 1—12 als Antifett-Crème.

17. Verwendung eines Präparates gemäss einem der Ansprüche 1—12 als Haarkur gegen fettes Haar.

18. Verfahren zur Hemmung der Sekretion der Talgdrüsen, dadurch gekennzeichnet, dass man ein Präparat gemäss einem der Ansprüche 1—12 auf die entsprechenden Hautpartien appliziert.

## Claims

1. Cosmetic preparation with antiseborrhoeic action containing pyridoxine tripalmitate, characterised in that it contains as the solubiliser an amine oxide of the general formula

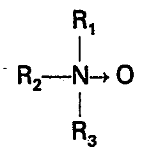

I

wherein $R_1$ signifies alkyl with 16—20 carbon atoms and $R_2$ and $R_3$ signify hydroxyethyl or methyl.

2. Preparation in accordance with claim 1, characterised in that it contains 0.1%—10% of an amine oxide of formula I.

3. Preparation in accordance with claim 1, characterised in that it contains 0.1%—2% of an amine oxide of formula I.

4. Preparation in accordance with claim 1, characterised in that it contains up to 50% alcohol.

5. Preparation in accordance with claim 1, characterised in that it contains up to 20% alcohol.

6. Preparation in accordance with one of claims 1—5, characterised in that it contains 0.2%—5% pyridoxine tripalmitate.

7. Preparation in accordance with one of claims 1—5, characterised in that it contains 1%—2% pyridoxine tripalmitate.

8. Preparation in accordance with one of claims 1—7, characterised in that it contains stearyldimethylamine oxide.

9. Preparation in accordance with one of claims 1—7, characterised in that it contains dimethylcetylamine oxide.

10. Preparation in accordance with one of claims 1—7, characterised in that it contains bis(hydroxyethyl)-stearinamine oxide.

11. Preparation in accordance with one of claims 1—7, characterised in that it contains dimethylhexadecylamine oxide.

12. Preparation in accordance with one of claims 1—7, characterised in that it contains hydrogenated dimethylstearinamine oxide.

13. Process for the manufacture of a cosmetic preparation in accordance with one of claims 1—12, characterised in that pyridoxine tripalmitate and a compound of formula I in claim 1, optionally in the presence of customary cosmetic adjuvants, are mixed while warming.

14. Use of a preparation in accordance with one of claims 1—12 as an anti-grease facial lotion.

15. Use of a preparation in accordance with one of claims 1—12 as an anti-grease shampoo.

16. Use of a preparation in accordance with one of claims 1—12 as an anti-grease cream.

17. Use of a preparation in accordance with one of claims 1—12 as a hair treatment against greasy hair.

18. Method for inhibiting the secretion of the sebaceous glands, characterised in that a preparation in accordance with one of claims 1—12 is applied to the corresponding skin areas.

**Revendications**

1. Une préparation cosmétique à action anti-séborrhéique contenant du tripalmitate de pyridoxine, caractérisée en ce qu'elle contient comme solubilisateur un aminoxyde de formula générale:

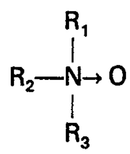

I

où $R_1$ représente un alcoyle ayant de 16 à 20 atomes de carbone, et $R_2$ et $R_3$ un hydroxyéthyle ou un méthyle.

2. Une préparation selon la revendication 1, caractérisée en ce qu'elle contient de 0,1 à 10% d'un aminoxyde de formula I.

3. Une préparation selon la revendication 1, caractérisée en ce qu'elle contient 0,1 à 2% d'un aminoxyde de formule I.

4. Une préparation selon la revendication 1, caractérisée en ce qu'elle contient jusqu'à 50% d'alcool.

5. Une préparation selon la revendication 1, caractérisée en ce qu'elle contient jusqu'à 20% d'alcool.

6. Une préparation selon l'une des revendications 1 à 5, caractérisée en ce qu'elle contient de 0,2 à 5% de tripalmitate de pyridoxine.

7. Une préparation selon l'une des revendications 1 à 5, caractérisée en ce qu'elle contient de 1 à 2% de tripalmitate de pyridoxine.

8. Une préparation selon l'une des revendications 1 à 7, caractérisée en ce qu'elle contient du stéaryldiméthylaminoxyde.

9. Une préparation selon l'une des revendications 1 à 7, caractérisée en ce qu'elle contient du diméthylcétylaminoxyde.

10. Une préparation selon l'une des revendications 1 à 7, caractérisée en ce qu'elle contient du bis(hydroxyéthyl)-aminoxyde de stéarine.

11. Une préparation selon l'une des revendications 1 à 7, caractérisée en ce qu'elle contient du diméthylhexadécylaminoxyde.

12. Une préparation selon l'une des revendications 1 à 7, caractérisée en ce qu'elle contient du diméthylaminoxyde de stéarine hydrogéné.

13. Un procédé de préparation d'une préparation cosmétique selon l'une des revendications 1 à 12, caractérisé en ce que l'on mélange en chauffant du tripalmitate de pyridoxine et un composé de formule I de la revendication 1, éventuellement en présence d'adjuvants cosmétiques habituels.

14. L'application d'une préparation selon l'une des revendications 1 à 12, comme lotion faciale contre la graisse.

15. L'application d'une préparation selon l'une des revendications 1 á 12, comme shampooing contre la graisse.

16. L'application d'une préparation selon l'une des revendications 1 à 12, comme crème contre la graisse.

17. L'application d'une préparation selon l'une des revendications 1 à 12, comme traitement contre les cheveux gras.

18. Un procédé d'inhibition de la sécrétion des glandes sébacées, caractérisé en ce que l'on applique une préparation selon l'une des revendications 1 à 12 sur les parties correspondantes de la peau.